# EUROPEAN PATENT SPECIFICATION

(11) **EP 1 790 221 B1**
(45) Date of publication and mention of the grant of the patent: **11.11.2009**
(21) Application number: 05783695.9
(22) Date of filing: 09.09.2005
(51) Int. Cl.: A61L 9/12, A01M 1/20

(54) **MEDICINE VOLATILIZER**
MEDIKAMENTENVERDUNSTER
DISPOSITIF DE VOLATILISATION DE MEDICAMENT

(30) Priority: 13.09.2004 JP 2004265751; 12.08.2005 JP 2005233853
(43) Date of publication of application: 30.05.2007
(73) Proprietor: DAINIHON JOCHUGIKU CO., LTD., Osaka-shi Osaka 550-0001 (JP)
(72) Inventor: KATSUDA, Yoshio, Dainihon Jochugiku Co., Ltd., Toyonaka-shi, Osaka 561-0827 (JP); IWASHIRO, Takeo, Kobe-shi, Hyogo 653-0022 (JP); KANZAKI, Tsutomu, Dainihon Jochugiku Co., Ltd., Toyonaka-shi, Osaka 561-0827 (JP)
(74) Representative: Hano, Christian
(86) International application number: PCT/JP2005/017102
(87) International publication number: WO 2006/030882

(56) References cited:
- EP-A- 1 184 499
- EP-A- 1 344 856
- JP-A- 2001 200 239
- JP-A- 2001 200 239
- JP-A- 2001 247 406
- JP-A- 2001 247 406
- JP-A- 2001 292 679
- US-A- 5 230 837

## Description

### TECHNICAL FIELD

The present invention relates to a chemical volatilization device according to the preamble of claim 1.

### BACKGROUND ART

EP1344856 discloses a double knitted fabric comprising of the front and rear webs and the linking yam linking both the webs, which contain mesh openings, and where the linking yam is knotted in two-needle stitch on at least one of the front and rear webs.

Japanese Unexamined Patent Publication No. 2001-247406, which is an application of the present applicant, advocates a chemical volatilization method for efficiently volatilizing and releasing a chemical by rotating a cartridge housing a granular chemical-impregnated body with a motor, and using the centrifugal force thereof and wind force generated by a fan. Although the constitution according to this method is extremely useful as a result of having superior volatilization performance and pest control effects, it has the shortcomings of requiring a rotating body in the form of a cartridge which houses a chemical-impregnated body, and not having a simple structure particularly in applications involving protecting clothing from pests, such as in dressers and closets.

In consideration of this situation, in the earlier application, the applicant has previously filed with this office a chemical volatilization device comprising impregnating a chemical into a plate-like body having gaps through which air can pass during rotation, and providing a rotary drive device for a rotating body, which is formed by one or a plurality of chemical retainers in the form of said plate-like body (Japanese Patent Application No. 2003-102369, and Japanese Patent Application No. 2004-095479, based on the priority claimed in accordance with Article 41, Paragraph 1 of the Patent Law on the basis of said application).

The invention of the aforementioned earlier-filed application is superior to the invention of Japanese Unexamined Patent Publication No. 2001-247406 in that it has a simpler constitution and can be used more easily.

In the plate-like chemical retainer serving as the subject of rotation in the invention of the aforementioned earlier-filed application, a constitution is connoted which forms a mesh in three-dimensional directions, and a chemical retainer employing such a constitution has superior ventilation.

However, in the aforementioned chemical retainer, in addition to requiring superior ventilation, it is also required to efficiently improve chemical volatilization effects.

Japanese Unexamined Patent Publication No. 2001-200239, which was filed by another firm, discloses the constitution of a chemical retainer comprising the overlapping of a plurality of nets composed of twisted threads, and sets forth that said constitution is superior to the case of a constitution employing a single net in terms of the retained amount of chemical and the amount of volatilized chemical.

However, in the aforementioned constitution as well, the only effects which are obtained are those in terms of retained amount of chemical and the amount of volatized chemical which are proportional to the number of nets, and a fundamental technical idea is not disclosed in the manner of improving chemical volatilization function in particular based on a laminated constitution formed by gaps between chemical-retaining fibers in the form of a net and other chemical-retaining fibers.

### DISCLOSURE OF THE INVENTION

An object of the present invention is to provide a constitution of a chemical volatilization device having a chemical retainer which, in addition to having a chemical retaining function based on an arrangement of mesh-like chemical-retaining fibers and other chemical-retaining fibers, is also able to improve chemical volatilization effects.

In order to solve the aforementioned problems, the basic constitution of the present invention is comprised of the features according to to claim 1.
(1) a chemical volatilization device for rotating a chemical retainer made of fibers as a material with a rotary drive device based on employing a chemical retainer, wherein, together with arranging chemical-retaining fibers in the form of a regular mesh in two-dimensional directions (hereinafter simply referred to as "mesh-like chemical-retaining fibers") on both the upper and lower sides of the chemical retainer, a plurality of chemical-retaining fibers are arranged between the mesh-like chemical-retaining fibers on the upper and lower sides formed in individual mesh units, which support and connect the chemical-retaining fibers on both the upper and lower sides at a predetermined interval (hereinafter simply referred to as "supportive connecting chemical-retaining fibers") as a result of having bending elasticity;
(2) the chemical volatilization device described in (1) above, wherein the mesh-like chemical-retaining fibers are in the form of twisted threads;
(3) the chemical volatilization device described in (1) or (2) above, wherein the supportive connecting chemical-retaining fibers form a columnar structure as a result of being arranged roughly in parallel in the vertical direction;
(4) the chemical volatilization device described in (1) or (2) above, wherein the supportive connecting chemical-retaining fibers form a diagonal structure as a result of being arranged in the state of intersecting on an angle in the vertical direction;
(5) the chemical volatilization device described in (4) above, wherein the diagonal structure is formed so as to connect sides or apices together located on the same side based on all four directions in mesh units corresponding to the upper and lower sides;
(6) the chemical volatilization device described in (4) above, wherein the diagonal structure is formed so as to connect sides or apices together located on opposite sides based on all four directions in mesh units corresponding to the upper and lower sides;
(7) the chemical volatilization device described in (1) or (2) above, wherein the supportive connecting chemical-retaining fibers form a columnar structure by being arranged roughly in parallel in the vertical direction, and form a diagonal structure by being arranged in the state of intersecting on an angle in the vertical direction;
(8) the chemical volatilization device described in (7) above, wherein the diagonal structure is formed so as to connect sides or apices together located on the same side based on all four directions in mesh units corresponding to the upper and lower sides;
(9) the chemical volatilization device described in (7) above, wherein the diagonal structure is formed so as to connect sides or apices together located on opposite sides based on all four directions in mesh units corresponding to the upper and lower sides;
(10) the chemical volatilization device described in (1) above,
   wherein small gap chemical-retaining fibers, which have a smaller gap than the mesh, and which are connected to the mesh-like chemical-retaining fibers on both sides, are arranged between the mesh-like chemical-retaining fibers on the upper and lower sides;
(11) the chemical volatilization device described in (1) above, wherein a plurality of chemical retainers consisting of the mesh-like chemical-retaining fibers arranged on the upper and lower sides and the supportive connecting chemical-retaining fibers arranged therebetween are overlapped;
(12) the chemical volatilization device described in (3) above,
   wherein the distance between the mesh-like chemical-retaining fibers on both sides is 1.0 to 10.0 mm; and
(13) the chemical volatilization device described in (1) above,
wherein the chemical retainer is housed by a protective case, which surrounds the upper and lower sides of the chemical retainer with an upper portion and lower portion, respectively and surrounds the outer circumference with a plurality of retaining frames, and of which a bearing located in the center is able to engage with a rotating shaft of the rotary drive device.

### BRIEF DESCRIPTION OF THE DRAWINGS

Fig. 1 shows the basic constitution of the chemical retainer of the present invention, with (a) being a planar view from above, (b) being a longitudinal cross-sectional view at a specific location in the planar view, and (c) being a planar view from below.
Fig. 2 shows the constitution of an embodiment of the present invention in which the unit shape of each mesh is different on the upper and lower sides, with (a) being a planar view from above, (b) being a longitudinal cross-sectional view at a specific location in the planar view, and (c) being a planar view from below.
Fig. 3 shows the constitution of the aforementioned (3), with (a) being a planar view, and (b) being a longitudinal cross-sectional view at a specific location in the planar view.
Fig. 4 shows the case where sides in the constitution of the aforementioned (5) are connected together by a diagonal structure, with (a) being a planar view, and (b) being a longitudinal cross-sectional view at a specific location in the planar view.
Fig. 5 shows the case where apices in the constitution of the aforementioned (5) are connected together by a diagonal structure, with (a) being a planar view, and (b) being a longitudinal cross-sectional view at a specific location in the planar view.
Fig. 6 shows the case where sides in the constitution of the aforementioned (6) are connected together by a diagonal structure, with (a) being a planar view, and (b) being a longitudinal cross-sectional view at a specific location in the planar view.
Fig. 7 shows the case where apices in the constitution of the aforementioned (6) are connected together by a diagonal structure, with (a) being a planar view, and (b) being a longitudinal cross-sectional view at a specific location in the planar view.
Fig. 8 shows the case where sides and apices in the constitution of the aforementioned (8) are connected together by a diagonal structure, with (a) being a planar view, and (b) being a longitudinal cross-sectional view at a specific location in the planar view.
Fig. 9 shows the case where sides and apices in the constitution of the aforementioned (9) are connected together by a diagonal structure, with (a) being a planar view, and (b) being a longitudinal cross-sectional view at a specific location in the planar view.
Fig. 10 is a cross-sectional view showing the constitution of the aforementioned (10) (showing the case where the supportive connecting chemical-retaining fibers employing a columnar structure as shown in Fig. 3 are used for the supportive connecting chemical-retaining fibers).
Fig. 11 is a perspective view of a protective case in which a chemical retainer is housed and which has been exploded into a cover portion located on the upper side of the chemical retainer, and a chemical retainer holder located on the lower side of the chemical retainer.

### EFFECT OF THE INVENTION

The chemical volatilization device according to the present invention is extremely useful as a result of not only having a chemical retaining function based on the arrangement of mesh-like chemical-retaining fibers and other chemical-retaining fibers, but also being able to improve chemical volatilization effects, and since it also has a simple structure, the device is extremely advantageous in terms of production cost as well.

### BEST MODE FOR CARRYING OUT THE INVENTION

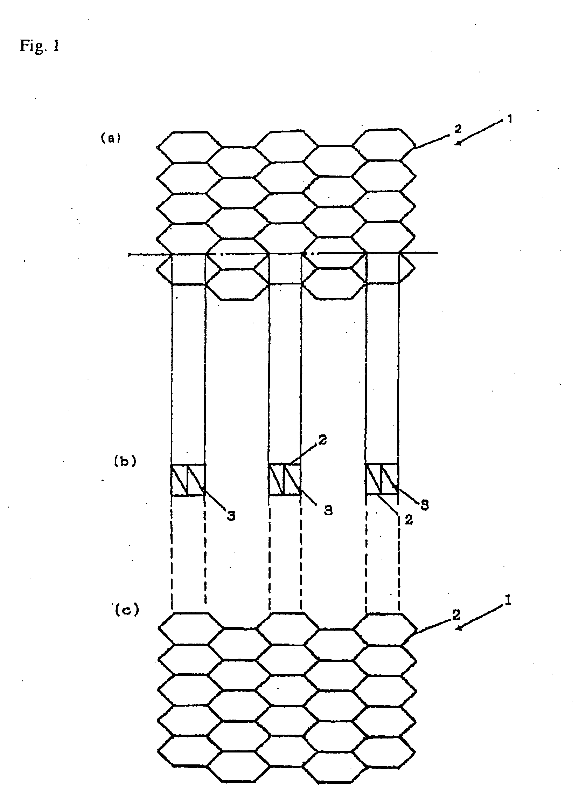

In the present invention, the basic constitution employs a chemical retainer 1, having for its basic constituent unit mesh-like chemical-retaining fibers 2, arranged on both the upper and lower sides, and supportive connecting chemical-retaining fibers 3, arranged between both the upper and lower sides, as a subject of rotation of a rotary drive device, as shown in Figs. 1(a), 1(b) and 1(c).

In Figs. 1(a) and 1(c), although the case is shown where the shape of the mesh of the mesh-like chemical-retaining fibers 2 is hexagonal, other shapes such as a rectangular shape or a triangular shape may naturally also be employed for the shape of said mesh.

In Figs. 1(a), 1(b) and 1(c), although the case is shown where mesh units on both the upper and lower sides are formed at roughly the same locations in the horizontal direction, and the mesh units on both the upper and lower sides have the same shape, embodiments of the present invention include not only such a case, but also the case where the mesh units on both the upper and lower sides are arranged in the state of deviating (being shifted) by a predetermined width in the horizontal direction, and the case where the shapes of the mesh units on both the upper and lower sides are mutually different (for example, the case where the shape of one of the mesh units is hexagonal while the shape of the other mesh unit is rectangular, and the case where, although both mesh units are of the same shape, e.g., the lower mesh unit shape is smaller than the upper mesh unit shape).

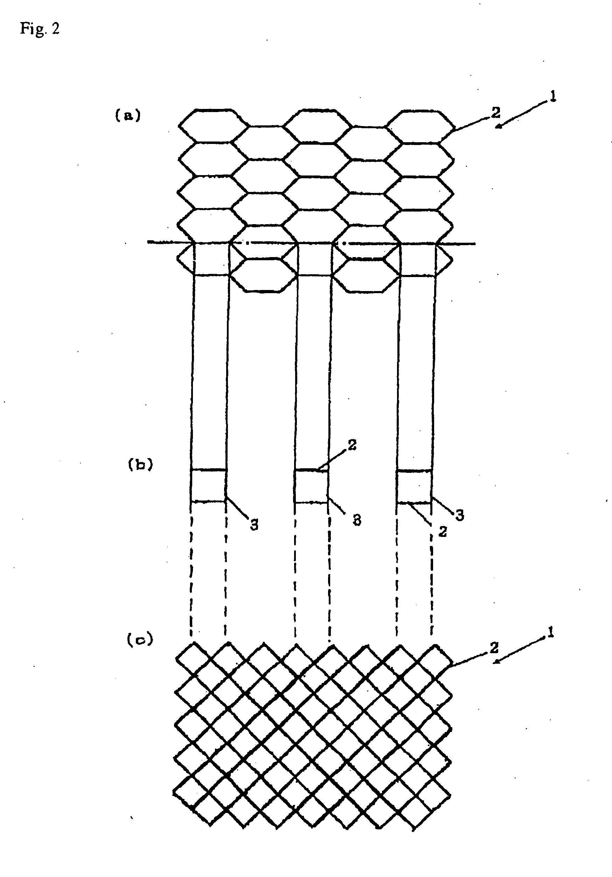

Furthermore, an aforementioned case where the shapes of the upper and lower mesh units are different is as shown in Figs. 2 (a), 2(b) and 2(c).

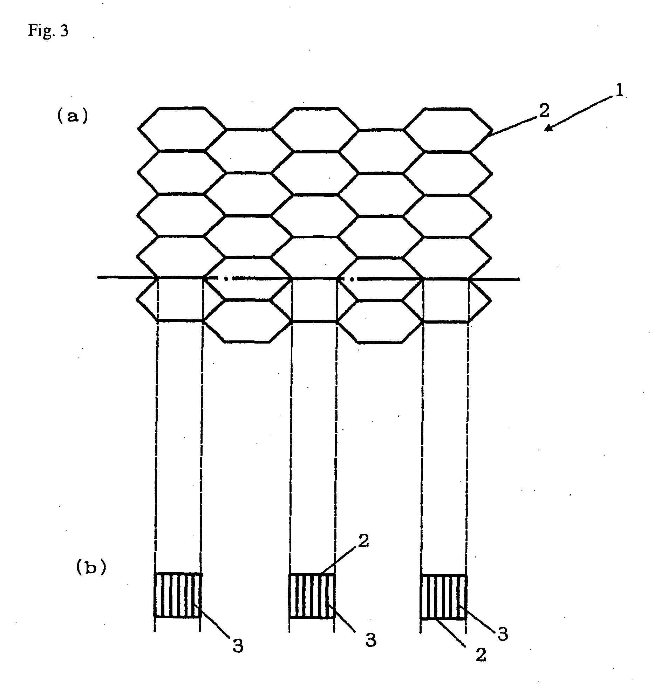

In Fig. 1(b), although fibers in the vertical direction and fibers intersecting on an angle with the vertical direction are respectively employed for supportive connecting chemical-retaining fibers 3, the supportive connecting form of these supportive connecting chemical-retaining fibers 3 is not limited to such a form, but rather a form in any direction may be employed provided the upper and lower mesh-like chemical-retaining fibers 2, which form each mesh unit, are supported and connected by a plurality of fibers by means of bending elasticity. (Furthermore, typical embodiments will be described later with reference to Figs. 3, 4, 5, 6 and 7.)

In the aforementioned basic constituent unit, turbulence accompanying negative pressure is generated in association with the rotational movement of each mesh-like chemical-retaining fiber 2 behind the direction of rotation of the mesh-like chemical-retaining fibers 2, and in association with this generation of negative pressure, air currents are generated in the horizontal direction from the gap regions of the supportive connecting chemical-retaining fibers 3 and towards upper and the lower mesh-like chemical-retaining fibers 2, and in the supportive connecting chemical-retaining fibers 3, chemical retained on the basis of capillary phenomena moves toward the mesh-like chemical-retaining fibers 2, resulting in augmentation of volatilization effects, while volatilization effects resulting from centrifugal force based on this rotation are also considered to be added thereto (it is not possible to presume a basis for favorable volatilization effects other than the aforementioned generation of negative pressure and generation of air currents).

As shown, for example, in Table 1 of Embodiment 3 to be described later, although these volatilization effects brought about by the aforementioned coupled constitution have been confirmed to be much more favorable than volatilization effects in the case of rotating only the mesh-like chemical-retaining fibers 2 or the mesh-like chemical-retaining fibers 2 obtained by overlapping two sheets, the specific mechanisms of the generation of turbulence and negative pressure are not completely clarified.

In order for mesh-like chemical-retaining fibers 2 in two-dimensional directions to have a satisfactory retaining function, the fibers should be in the form of twisted threads as described in (2) above. Moreover, by increasing the number of threads that compose the twisted threads, gaps should be formed which make it possible to retain a chemical based on capillary phenomena.

The material which composes the aforementioned mesh-like chemical-retaining fibers 2 and supportive connecting chemical-retaining fibers 3 is preferably that which does not affect the stability of the chemical and demonstrates constant and continuous volatilization. Examples of materials which can be used include natural fibers such as cotton, hemp, wool and silk, semi-synthetic fibers such as rayon, synthetic fibers such as polyester, nylon, acrylic, vinylon, polyethylene, polypropylene, aramid, polyethylene naphthalate and polyphenylene sulfide, and inorganic fibers such as glass fibers, carbon fibers and ceramic fibers.

Since the supportive connecting chemical-retaining fibers 3 are coupled with the mesh-like chemical-retaining fibers 2 in each mesh-like constituent unit, said supportive connecting chemical-retaining fibers 3 compose fine gaps in the horizontal direction, and have the function of retaining a chemical based on capillary phenomena in said gaps.

Thus, the gaps between the supportive connecting chemical-retaining fibers 3 are required to be in such a degree which allows the creation of a retaining function resulting from the aforementioned capillary phenomena.

A typical example of the supportive connecting chemical-retaining fibers 3 is described in (3) above, and as shown in Fig. 3, an embodiment in which the supportive connecting chemical-retaining fibers 3 are arranged roughly in parallel in the vertical direction to form a columnar structure offers advantages in terms of having a comparatively simple structure and being easily produced.

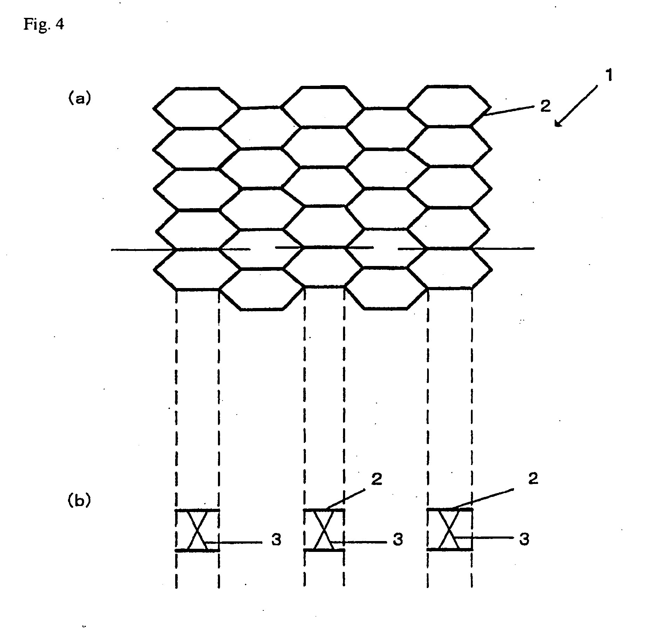

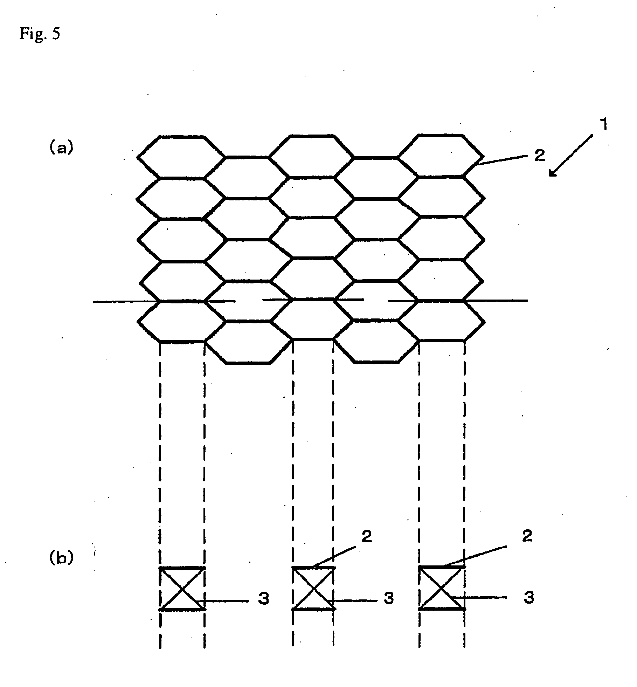

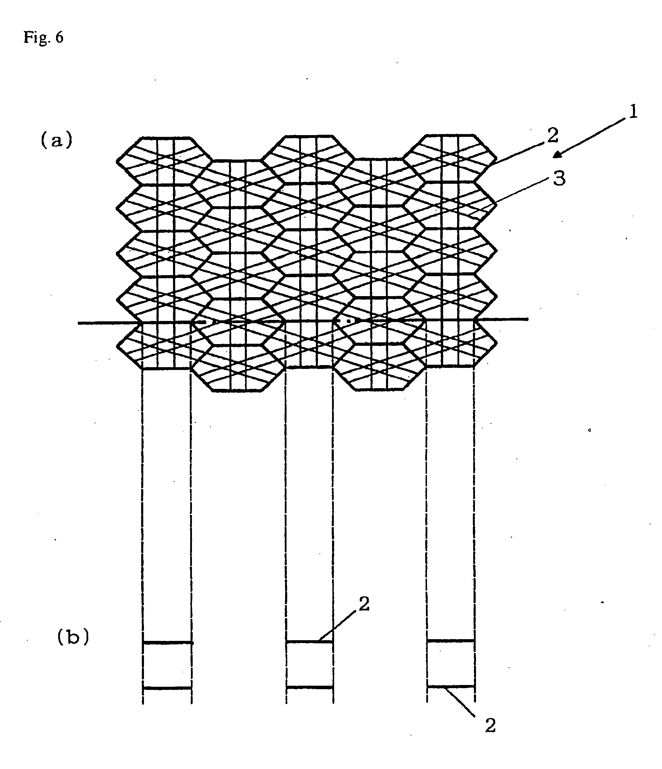

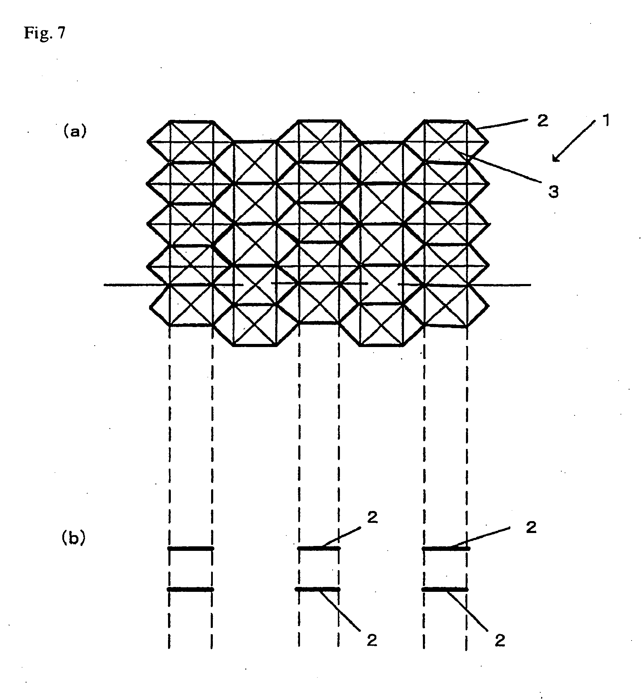

In contrast, in the case of a diagonal constitution described in (4) above, as shown in Figs. 4 and 5, although a diagonal constitution is typically realized in the form of the constitution according to (5) above, in which the diagonal structure is formed so as to connect sides or apices together located on the same side based on all four directions in mesh units corresponding to the upper and lower sides as shown in Figs. 4 and 5, or a constitution according to (6) above, in which the diagonal structure is formed so as to connect sides or apices together located on opposite sides based on all four directions in mesh units corresponding to the upper and lower sides as shown in Figs. 6 and 7, these constitutions demonstrate a function like that of reinforcing beams in the diagonal directions of an architectural structure, and together with enabling the upper and lower mesh-like chemical-retaining fibers 2 and the supportive connecting chemical-retaining fibers 3 therebetween to be securely coupled, as a result of forming the fibers in the diagonal direction, provide the additional advantage of also enabling this to serve as a chemical retaining function.

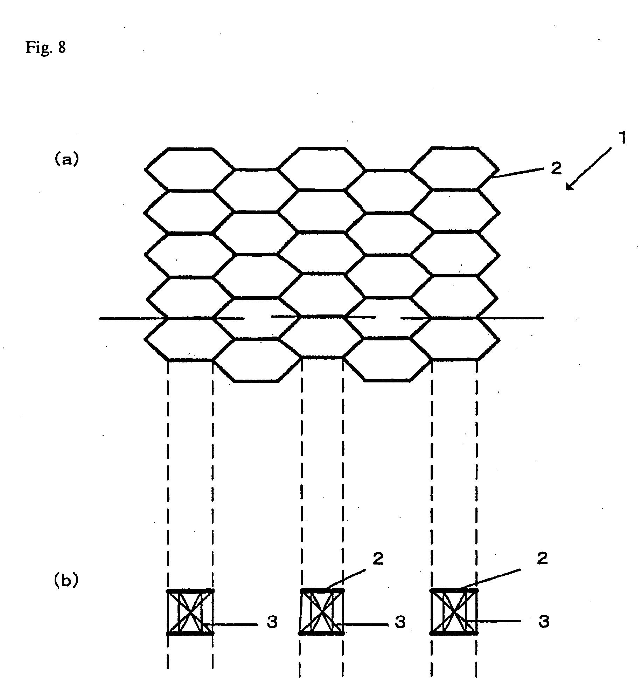

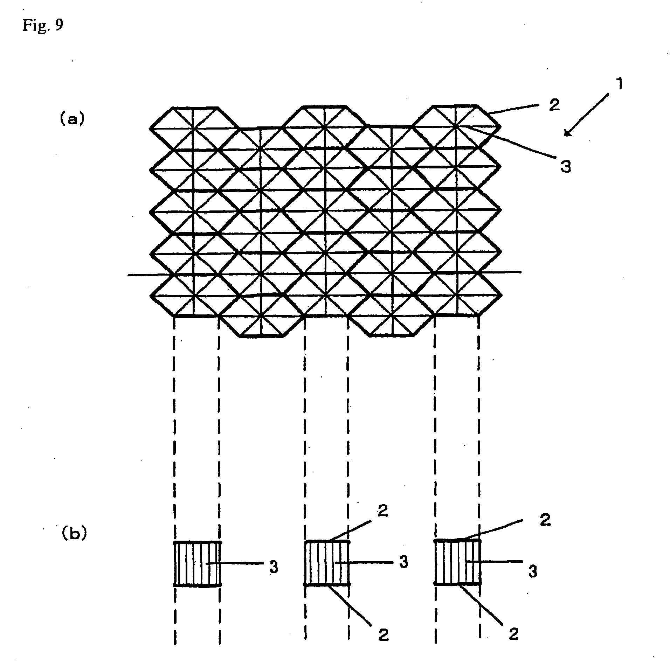

Since the constitution according to (7) above is a combination of the constitution of (3) above and the constitution of (4) above, although this constitution is typically realized in the form of the constitution according to (8) above corresponding to the aforementioned (5) as shown in Fig. 8 (with Fig. 8 indicating the use of a diagonal structure in which sides and apices are respectively connected), and in the form of the constitution of (9) above corresponding the aforementioned (6) as shown in Fig. 9 (with Fig. 9 indicating a constitution in which a portion of the sides and a portion of the apices are connected together), these constitutions offer the advantages of the constitution of (3) above and the constitution of (4) above, and together with enabling the retained state in the vertical direction to be even stronger, provide the additional advantage of also enabling this to serve as a chemical retaining function.

The constitution of (4) above is not limited to the constitutions according to (5) and (6) above, and similarly, the constitution of (7) above is not limited to the constitutions according to (8) and (9) above.

Although each side is connected to a corresponding side and each apex is connected to a corresponding apex in Figs. 4 and 5 relating to (5) above, in Figs. 6 and 7 relating to (6) above, in Fig. 8 relating to (8) above, and in Fig. 9 relating to (9) above, the constitutions of (4) and (7) above are not limited to these constitutions, but rather a constitution which connects each side with each apex can naturally also be realized, and in such a connected state as well, the above-mentioned advantages can be demonstrated.

Furthermore, in Figs. 3, 4, 5, 6, 7, 8 and 9, since the shape of each upper and lower mesh is the same and is located in the same direction, the bottom planar views as in Fig. 1(c) and Fig. 2(c) have been omitted.

Embodiments of the supportive connecting chemical-retaining fibers 3 are not limited to the cases of (3) and (4) above, but rather, embodiments can also be employed in which, for example, said fibers are randomly arranged in the vertical direction.

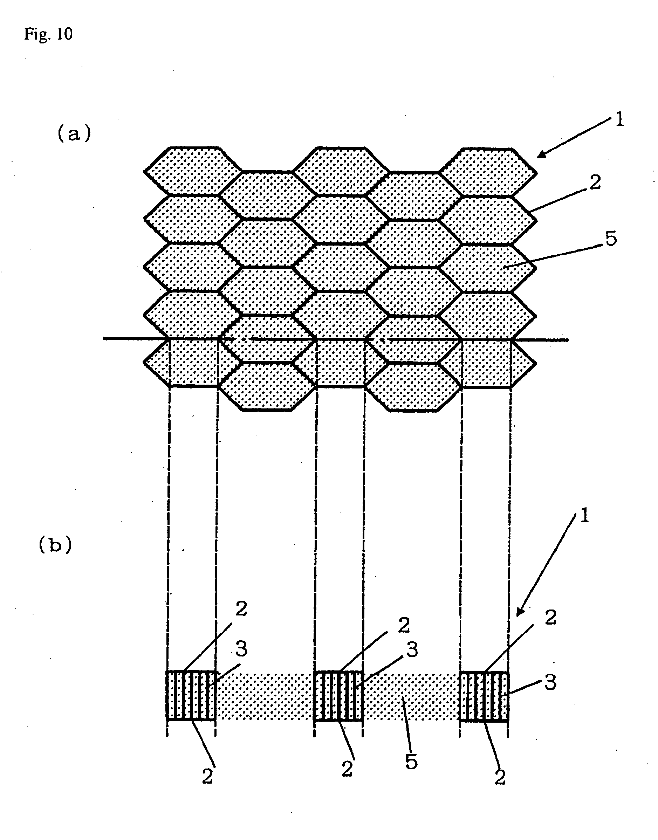

As shown in Figs. 3, 4, 5, 6, 7, 8 and 9, although it is possible to form the present invention with chemical retainer 1, mesh-like chemical-retaining fibers 2 and supportive connecting chemical-retaining fibers 3, in addition to this type of embodiment, an embodiment can also be employed by arranging small gap chemical-retaining fibers 5, which have gaps smaller than the mesh and are connected to the mesh-like chemical-retaining fibers 2 of both sides, between mesh-like chemical-retaining fibers 2 on both the upper and lower sides, as described in the aforementioned (10) and as shown in Fig. 10.

Although the chemical retaining function can be increased in the embodiment of (10) above, in said embodiment, volatilization effects are to be demonstrated as a result of air moving towards the mesh-like chemical-retaining fibers 2 on the upper and lower sides through the gaps of the small gap chemical-retaining fibers 5.

In the present invention, although the chemical retainer 1 is formed based on a constituent unit comprised of the mesh-like chemical-retaining fibers 2 arranged on both the upper and lower sides and the supportive connecting chemical-retaining fibers 3 arranged between the upper and lower sides, as shown in Fig. 1, in addition to an embodiment in which the aforementioned constituent unit is employed as a single unit, an embodiment can also be employed in which a plurality of the chemical retainers 1, comprised of the mesh-like chemical-retaining fibers 2 arranged on both the upper and lower sides and the supportive connecting chemical-retaining fibers 3 arranged therebetween, are overlapped as described in the aforementioned (11).

Adequate chemical retaining function can be demonstrated based on a laminated constitution in the embodiment described in (11) above.

The upper and lower widths of the constituent unit comprised of mesh-like chemical-retaining fibers arranged on both the upper and lower sides and supportive connecting chemical-retaining fibers arranged between both the upper and lower sides are influenced by the surface area of each unit of the mesh-like chemical-retaining fibers 2 and the density in the horizontal direction of the supportive connecting chemical-retaining fibers 3, and although the distance is to be set larger, the greater the surface area of the mesh form of each unit is and the smaller the density of the supportive connecting chemical-retaining fibers 3 is, it is normally set to within a range of 1.0 to 10.0 mm.

There are no particular limitations on the shape of the chemical retainer 1, and the shape may be suitably determined corresponding to the purpose, examples of which include a disc-like shape, doughnut shape, gear shape or fan shape. Since the intent of the present invention is to provide a chemical retainer 1 which is applied to a small chemical volatilization device, if the shape of the chemical retainer 1 is a disc-like shape, for example, specifications based on an outer diameter of about 3 to 5 cm is advantageous in terms of use.

Examples of chemicals used in the present invention include volatile insecticides, miticides, pesticides, aromatic agents and deodorizers.

Pyrethroid-based chemicals, which are volatile at normal temperatures, are preferable for the insecticide, and example of such a chemical is the fluorine-substituted benzyl alcohol ester compound represented by general formula (I): 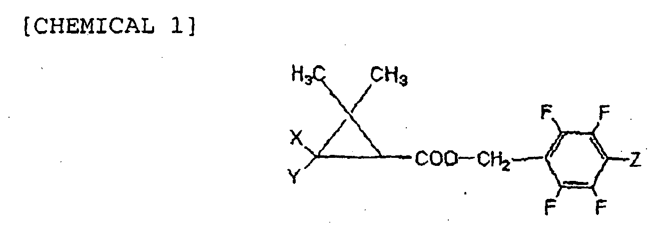 (wherein, X represents a hydrogen atom or methyl group, Y represents a vinyl group, 1-propenyl group, 2-methyl-1-propenyl group, 2,2-dichlorovinyl group, 2,2-difluorovinyl group or 2-chloro-2-trifluoromethylvinyl group when X is a hydrogen atom, Y represents a methyl group when X is a methyl group, and Z represents a hydrogen atom, fluorine atom, methyl group, methoxymethyl group or propargyl group).

Specific examples of compounds represented by general formula (I) include, but are not limited to, 2,3,5,6-tetrafluorobenzyl-2,2-dimethyl-3-(2,2-dichlorovinyl) cyclopropane carboxylate (hereinafter referred to as Compound A), 4-methyl-2,3,5,6-tetrafluorobenzyl-2,2-dimethyl-3-(1-propenyl)cyclopropane carboxylate (hereinafter referred to as Compound B), 4-methoxymethyl-2,3,5,6-tetrafluorobenzyl-2,2-dimethyl-3-(1-propenyl)cyclopropane carboxylate (hereinafter referred to as Compound C), 4-propargyl-2,3,5,6-tetrafluorobenzyl-2,2,3,3-tetramethylcyclopropane carboxylate (hereinafter referred to as Compound D), and 4-methoxymethyl-2,3,5,6-tetrafluorobenzyl-2,2,3,3-tetramethylcyclopropane carboxylate (hereinafter referred to as Compound E). One type of a compound represented by general formula (I) may be used, or two or more types of the compounds may be used in combination. Furthermore, although the compounds represented by general formula (I) include optical isomers and geometrical isomers based on asymmetric carbons and double bonds, each of these along with arbitrary mixtures thereof are naturally also included in the present invention.

Although depending on the type of chemical, expiration date, size of chemical retainer 1 and so forth, the amount of chemical retained in the chemical retainer 1 is suitably set to about 20 to 400 mg in the case where the chemical is, for example, the aforementioned pyrethroid-based chemical which is volatile at normal temperatures.

A solvent, diluent, surfactant, dispersant or slow-release agent and so forth can be used as needed when retaining the chemical, and various means known in the prior art can be used. Moreover, a stabilizer, fragrance, colorant or antistatic agent and so forth can also be blended into the chemical appropriately.

An ordinary motor based on an alternating current power supply or direct current power supply is used for the rotary drive device, and the rotating speed thereof should be 500 to 2000 rpm.

The basis for employing a rotating speed within the aforementioned range is that, in the case where the rotating speed is less than 500 rpm, the chemical volatilization performance deteriorates, while if the rotating speed exceeds 2000 rpm, there is the risk of the chemical being scattered from retainer 1 during rotation.

The following provides an explanation of the present invention in accordance with embodiments thereof.

### (EMBODIMENT)

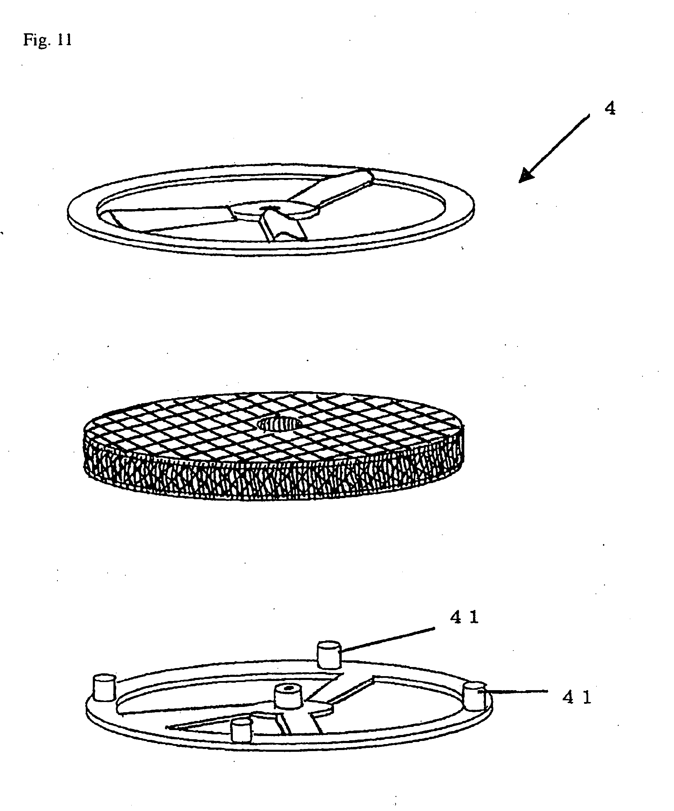

As shown in Fig. 11, this embodiment is characterized by housing a chemical retainer 1 with a protective case 4, which surrounds the upper and lower sides of chemical retainer 1 by upper side and lower side portions, respectively, surrounds the outer circumference with a plurality of retaining frames 41, and is able to engage with a rotating shaft of a rotary drive device at a central location.

In this embodiment, the chemical retainer 1 is housed in the state of being surrounded on both the upper and lower sides and the circumference thereof by the protective case 4, and since the protective case 4 engages with the rotating shaft of the rotary drive device, the protective case 4 and the chemical retainer 1 are to rotate as a single unit during rotation.

The use of protective case 4 is advantageous in the case of being unable to obtain stable rotation as a result of supporting the rotating shaft of the rotary drive device with chemical retainer 1 alone since chemical retainer 1 has an easily deformable shape, while also making it possible to demonstrate the function of preventing physical contact with the chemical with hands or fingers.

Although there are no particular limitations on the shape or numbers of retaining frames 41, in consideration of design convenience, it is suitable to provide a plurality of the retaining frames 41 having, for example, a plate-like shape for the upper and lower sides, and a shape having a circular, triangular or square cross-section for the circumference.

When housing the chemical retainer 1 in the protective case 4, although chemical retainer 1 can naturally be housed after retaining a chemical, a method of placing the chemical retainer 1 in the protective case 4 before retaining the chemical, and then dispensing the chemical with the cover member removed, followed by closing the cover member is advantageous in terms of production.

Experiment results conforming to this type of embodiment in which a chemical retainer 1 is housed in a protective case 4 are explained as indicated below.

### [Experiment Example 1]

Two-dimensional mesh-like chemical-retaining fibers 2 were woven on both the upper and lower sides using twisted polyester fibers, and supportive connecting chemical-retaining fibers 3 (upper and lower widths: 3 mm), comprised of polyester fibers, were arranged between both sides based on a diagonal structure according to the constitution of the aforementioned (6) as shown in Fig. 6 to produce a disc-like chemical retainer 1 having an overall thickness in the vertical direction of 4.0 mm and an outer diameter of 4.0 cm.

The chemical retainer 1 was housed in a protective case 4 made of polycarbonate (outer diameter: 5.0 cm, thickness: 8 mm), and the protective case 4 employed a roughly circular cross-section for circumferential retaining frames 41.

In this type of chemical retainer 1, a chemical solution, in which 40 mg of Compound C (4-methoxymethyl-2,3,5,6-tetrafluorobenzyl-2,2-dimethyl-3-(1-propenyl)cyclopropane carboxylate) were dissolved in kerosene, was retained in said chemical retainer 1, and after housing in the protective case 4, was activated for 120 hours at a rotating speed of 1200 rpm.

During this activation, there was no scattering of the chemical from the chemical retainer 1, and mosquitoes were effectively controlled over the course of about 120 hours.

### [Experiment Example 2]

Two-dimensional mesh-like chemical-retaining fibers 2 were woven using twisted polyester fibers, and supportive connecting chemical-retaining fibers 3 (upper and lower widths: 2.5 mm), comprised of nylon fibers, were arranged in a state in which a columnar structure and a diagonal structure were overlapped based on the constitution of the aforementioned (8) shown in Fig. 8 to produce a disc-like chemical retainer 1 having an overall thickness in the vertical direction of 4.2 mm and an outer diameter of 4.5 cm.

70 mg of Compound A (2,3,5,6-tetrafluorobenzyl-2,2-dimethyl-3-(2,2-dichlorovinyl)cyclopropane carboxylate) was retained instead of the chemical used in Experiment Example 1 and activated for 240 hours.

This chemical retainer 1 was housed in the protective case 4 of Experiment Example 1, activated for 8 hours per day by suspending in a doghouse at a rotating speed of 1400 rpm, and said activation was continued for about 30 days.

During this continuous activation, the chemical volatilization device demonstrated stable insecticidal effects against harmful insects such as mosquitoes and midges, and the dog was free from the harmful insects.

### [Experiment Example 3]

After housing various types of chemical retainers 1 employing the constitution of (8) above shown in Fig. 8 in various types of protective cases 4 in compliance with Experimental Example 1, the devices were activated in a six-tatami mat room at a rotating speed of 1200 rpm, and insecticidal effects against adult mosquitoes were investigated immediately after the start and 120 hours after the start. A test of insecticidal effects was carried out by investigating the knockdown effect against mosquitoes exposed for 2 hours after releasing 100 adult male Culex pipiens in a six-tatami mat room during the activation of the device, and determining the KT₅₀ value. A chemical retainer 1 composed only of mesh-like chemical-retaining fibers 2 (the upper side is indicated with "F" while the lower side is indicated with "B" in the table) without weaving supportive connecting chemical-retaining fibers 3 (indicated with "M" in the table) (Control Example 1), and the case of combining two separate units of mesh-like chemical-retaining fibers (Control Example 2) were used as controls.

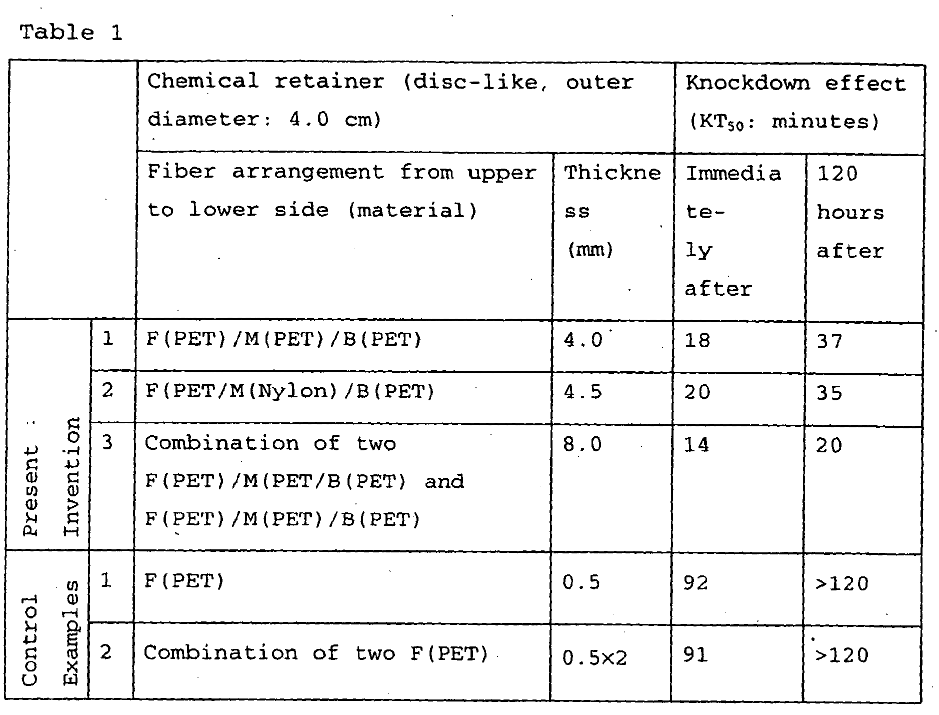

As a result of the test, the chemical volatilization devices according to the present invention were proved to stably maintain superior insecticidal effects over an extended time period of 120 hours. In the case of overlapping a plurality of fibers as described in the aforementioned (11), the chemical retainer 1 was also observed to demonstrate superior chemical volatilization effects.

However, since the thickness of chemical retainer 1 tends to increase in the aforementioned case of overlapping a plurality of fibers, it is necessary to give consideration to the design by taking account of both convenience of production and handling.

On the other hand, the chemical retainer 1 composed only of the mesh-like chemical-retaining fibers 2 (Control Example 1) and the case of combining two separate units of the mesh-like chemical-retaining fibers 2 (Control Example 2) were proved to have both poor chemical retaining performance and chemical volatilization performance as compared with the devices of the present invention, and were clearly proved to be inferior. As a result, effects in terms of chemical volatilization function resulting from coupling the mesh-like chemical-retaining fibers 2, arranged on both the upper and lower sides, and the supportive connecting chemical-retaining fibers 3, arranged between the upper and lower sides, were able to be confirmed to exist.

### INDUSTRIAL APPLICABILITY

The chemical volatilization device of the present invention is able to efficiently volatilize and release a chemical, thereby enabling it to also be applied in fields other than pest control, such as aromatics, deodorization and antimicrobial applications, after suitably selecting the active ingredient.

## Claims

1. A chemical volatilization device comprising:
- a chemical retainer (1), and
- a rotary drive device for rotating the chemical retainer (1)
wherein said chemical retainer (1) is housed by a protective case (4), which surrounds the upper and lower sides of the chemical retainer (1) with an upper portion and lowe portion, respectively, and surrounds the outer circumference of the chemical retainer (1) with a plurality of retaining frames (41),
wherein said protective case (4) has a bearing located in the center, which is able to engage with a rotating shaft of the rotary drive device.
**characterized in that**
said chemical retainer (1) is composed of
- mesh-like chemical-retaining fibers(2) in the form of regular mesh units in two-dimensional directions, on both the upper and lower sides of the chemical retainer (1), and
- a plurality of supportive connecting chemical-retaining fibers (3),
wherein said mesh-like chemical-retaining fibers (2) are in the form of twisted threads, and
wherein said supportive connecting chemical-retaining fibers (3) are arranged between the mesh-like chemical-retaining fibers (2), supporting and connecting the mesh-like chemical-retaining fibers (2) on both the upper and lower sides at a predetermined interval as a result of having bending elasticity,

2. The chemical volatilization device according to claim 1, wherein the supportive connecting chemical-retaining fibers (3) form a columnar structure as a result of being arranged roughly in parallel in the vertical direction.

3. The chemical volatilization device according to claim 1, wherein the supportive connecting chemical-retaining fibers (3) form a diagonal structure as a result of being arranged in the state of intersecting on an angle in the vertical direction.

4. The chemical volatilization device according to claim 3, wherein the diagonal structure is formed so as to connect sides or apices together located on the same side based on all four directions in mesh units corresponding to the upper and lower sides.

5. The chemical volatilization device according to claim 3, wherein the diagonal structure is formed so as to connect sides or apices together located on opposite sides based on all four directions in mesh units corresponding to the upper and lower sides.

6. The chemical volatilization device according to claim 1, wherein the supportive connecting chemical-retaining fibers (3) form a columnar structure by being arranged roughly in parallel in the vertical direction, and form a diagonal structure by being arranged in the state of intersecting on an angle in the vertical direction.

7. The chemical volatilization device according to claim 6, wherein the diagonal structure is formed so as to connect sides or apices together located on the same side based on all four directions in mesh units corresponding to the upper and lower sides.

8. The chemical volatilization device according to claim 6, wherein the diagonal structure is formed so as to connect sides or apices together located on opposite sides based on all four directions in mesh units corresponding to the upper and lower sides.

9. The chemical volatilization device according to claim 1, wherein small gap chemical-retaining fibers (5), which have a smaller gap than the mesh, and which are connected to the mesh-like chemical-retaining fibers (2) on both sides, are arranged between the mesh-like chemical-retaining fibers on the upper and lower sides.

10. The chemical volatilization device according to claim 1, wherein a plurality of chemical retainers (1) consisting of the mesh-like chemical-retaining fibers (2) arranged on the upper and lower sides and the supportive connecting chemical-retaining fibers (3) arranged therebetween are overlapped.

11. The chemical volatilization device according to claim 2, wherein the distance between the mesh-like chemical-retaining fibers (2) on both sides is 1.0 to 10.0 mm.

## Patentansprüche

1. Chemikalienverflüchtigungsvorrichtung umfassend:
- eine Chemikalienrückhalteeinrichtung (1) und
- eine Drehantriebsvorrichtung zum Drehen der Chemikalienrückhalteeinrichtung (1)
wobei die Chemikalienrückhalteeinrichtung (1) von einem Schutzgehäuse (4) aufgenommen ist, das die Ober- und die Unterseite der Chemikalienrückhalteeinrichtung (1) mit einem oberen Abschnitt bzw. einem unteren Abschnitt umgibt und den Außenumfang der Chemikalienrückhalteinrichtung (1) mit einer Vielzahl von Halterahmen (41) umgibt,
wobei das Schutzgehäuse (4) ein im Zentrum angeordnetes Lager aufweist, das mit einer Drehwelle der Drehantriebsvorrichtung in Eingriff kommen kann,
**dadurch gekennzeichnet, dass** die Chemikalienrückhalteeinrichtung (1) aus
- maschenartigen Chemikalienrückhaltefasern (2) in Form von regelmäßigen Mascheneinheiten in zweidimensionalen Richtungen auf der Ober- sowie auch auf der Unterseite der Chemikalienrückhalteeinrichtung (1) und
- einer Vielzahl von stützenden verbindenden Chemikalienrückhaltefasern (3) zusammengesetzt ist,
wobei die maschenartigen Chemikalienrückhaltefasern (2) die Form von verdrehten Fäden haben, und
wobei die stützenden verbindenden Chemikalienrückhaltefasern (3) zwischen den maschenartigen Chemikalienrückhaltefasern (2) angeordnet sind, wobei sie infolge dessen, dass sie Biegeelastizität aufweisen, die maschenartigen Chemikalienrückhaltefasern (2) auf der Ober- sowie auch auf der Unterseite in einem vorherbestimmten Intervall stützen und verbinden.

2. Chemikalienverflüchtigungsvorrichtung nach Anspruch 1, wobei die stützenden verbindenden Chemikalienrückhaltefasern (3) infolge dessen, dass sie in der Vertikalrichtung annähernd parallel angeordnet sind, einen Säulenaufbau bilden.

3. Chemikalienverflüchtigungsvorrichtung nach Anspruch 1, wobei die stützenden verbindenden Chemikalienrückhaltefasern (3) infolge dessen, dass sie in der Vertikalrichtung in dem Zustand angeordnet sind, in dem sie sich in einem Winkel schneiden, einen Diagonalaufbau bilden.

4. Chemikalienverflüchtigungsvorrichtung nach Anspruch 3, wobei der Diagonalaufbau so ausgebildet ist, dass er Seiten oder Spitzen miteinander verbindet, die sich in Mascheneinheiten, die der Ober- und der Unterseite entsprechen, auf der Basis aller vier Richtungen auf der gleichen Seite befinden.

5. Chemikalienverflüchtigungsvorrichtung nach Anspruch 3, wobei der Diagonalaufbau so ausgebildet ist, dass er Seiten oder Spitzen miteinander verbindet, die sich in Mascheneinheiten, die der Ober- und der Unterseite entsprechen, auf der Basis aller vier Richtungen auf gegenüberliegenden Seiten befinden.

6. Chemikalienverflüchtigungsvorrichtung nach Anspruch 1, wobei die stützenden verbindenden Chemikalienrückhaltefasern (3) einen Säulenaufbau bilden, indem sie in der Vertikalrichtung annähernd parallel angeordnet sind, und einen Diagonalaufbau bilden, indem sie in der Vertikalrichtung in dem Zustand angeordnet sind, in dem sie sich in einem Winkel schneiden.

7. Chemikalienverflüchtigungsvorrichtung nach Anspruch 6, wobei der Diagonalaufbau so ausgebildet ist, dass er Seiten oder Spitzen miteinander verbindet, die sich in Mascheneinheiten, die der Ober- und der Unterseite entsprechen, auf der Basis aller vier Richtungen auf der gleichen Seite befinden.

8. Chemikalienverflüchtigungsvorrichtung nach Anspruch 6, wobei der Diagonalaufbau so ausgebildet ist, dass er Seiten oder Spitzen miteinander verbindet, die sich in Mascheneinheiten, die der Ober- und der Unterseite entsprechen, auf der Basis aller vier Richtungen auf gegenüberliegenden Seiten befinden.

9. Chemikalienverflüchtigungsvorrichtung nach Anspruch 1, wobei Chemikalienrückhaltefasern (5) mit kleinem Spalt, die einen kleineren Spalt als die Masche aufweisen und die mit den maschenartigen Chemikalienrückhaltefasern (2) an beiden Seiten verbunden sind, zwischen den maschenartigen Chemikalienrückhaltefasern auf der Ober- und der Unterseite verbunden sind.

10. Chemikalienverflüchtigungsvorrichtung nach Anspruch 1, wobei eine Vielzahl von Chemikalienrückhaltereinrichtungen (1), die aus den maschenartigen Chemikalienrückhaltefasern (2) bestehen, die auf der Ober- und der Unterseite angeordnet sind, und die stützenden verbindenden Chemikalienrückhaltefasern (3), die dazwischen angeordnet sind, überlappend sind.

11. Chemikalienverflüchtigungsvorrichtung nach Anspruch 2, wobei der Abstand zwischen den maschenartigen Chemikalienrückhaltefasern (2) auf beiden Seiten 1,0 bis 10,0 mm beträgt.

## Revendications

1. Dispositif de volatilisation de produit chimique comprenant :
- un rétenteur chimique (1), et
- un dispositif d'entraînement rotatif pour mettre en rotation le rétenteur chimique (1)
dans lequel ledit rétenteur chimique (1) est reçu par un boîtier de protection (4), qui entoure les côtés supérieur et inférieur du rétenteur chimique (1) avec une partie supérieure et une partie inférieure, respectivement, et entoure la circonférence extérieure du rétenteur chimique (1) avec une pluralité de cadres de rétention (41),
dans lequel ledit boîtier de protection (4) a un palier situé au centre, qui est apte à s'engager avec un arbre rotatif du dispositif d'entraînement rotatif,
**caractérisé en ce que**
ledit rétenteur chimique (1) est composé de
- fibres de rétention de produit chimique en maillage (2) sous la forme d'unités de maillage régulières dans des directions bidimensionnelles, à la fois sur les côtés supérieur et inférieur du rétenteur chimique (1), et
- une pluralité de fibres de rétention de produit chimique de support et de connexion (3),
dans lequel lesdites fibres de rétention de produit chimique en maillage (2) sont sous la forme de fils torsadés, et
dans lequel lesdites fibres de rétention de produit chimique de support et de connexion (3) sont agencées entre les fibres de rétention de produit chimique en maillage (2), supportant et connectant les fibres de rétention de produit chimique en maillage (2) à la fois sur les côtés supérieur et inférieur à un intervalle prédéterminé en résultat de leur possession d'une élasticité en flexion.

2. Dispositif de volatilisation de produit chimique selon la revendication 1,
dans lequel les fibres de rétention de produit chimique de support et de connexion (3) forment une structure colonnaire en résultat de leur agencement grossièrement en parallèle dans la direction verticale.

3. Dispositif de volatilisation de produit chimique selon la revendication 1,
dans lequel les fibres de rétention de produit chimique de support et de connexion (3) forment une structure diagonale en résultat de leur agencement dans l'état d'intersection selon un angle dans la direction verticale.

4. Dispositif de volatilisation de produit chimique selon la revendication 3,
dans lequel la structure diagonale est formée de façon à connecter des côtés ou des sommets ensemble situés sur le même côté sur la base de la totalité des quatre directions dans des unités de maillage correspondant aux côtés supérieur et inférieur.

5. Dispositif de volatilisation de produit chimique selon la revendication 3,
dans lequel la structure diagonale est formée de façon à connecter des côtés ou des sommets ensemble situés sur des côtés opposés sur la base de la totalité des quatre directions dans des unités de maillage correspondant aux côtés supérieur et inférieur.

6. Dispositif de volatilisation de produit chimique selon la revendication 1,
dans lequel les fibres de rétention de produit chimique de support et de connexion (3) forment une structure colonnaire en étant agencées grossièrement en parallèle dans la direction verticale, et forment une structure diagonale en étant agencées dans l'état d'intersection selon un angle dans la direction verticale.

7. Dispositif de volatilisation de produit chimique selon la revendication 6,
dans lequel la structure diagonale est formée de façon à connecter des côtés ou des sommets ensemble situés sur le même côté sur la base de la totalité des quatre directions dans des unités de maillage correspondant aux côtés supérieur et inférieur.

8. Dispositif de volatilisation de produit chimique selon la revendication 6,
dans lequel la structure diagonale est formée de façon à connecter des côtés ou des sommets ensemble situés sur des côtés opposés sur la base de la totalité des quatre directions dans des unités de maillage correspondant aux côtés supérieur et inférieur.

9. Dispositif de volatilisation de produit chimique selon la revendication 1,
dans lequel des fibres de rétention de produit chimique à petit espace (5), qui ont un espace plus petit que le maillage, et qui sont connectées aux fibres de rétention de produit chimique en maillage (2) sur les deux côtés, sont agencées entre les fibres de rétention de produit chimique en maillage sur les côtés supérieur et inférieur.

10. Dispositif de volatilisation de produit chimique selon la revendication 1,
dans lequel une pluralité de rétenteurs de produit chimique (1) consistant en les fibres de rétention de produit chimique en maillage (2) agencées sur les côtés supérieur et inférieur et les fibres de rétention de produit chimique de support et de connexion (3) agencées entre celles-ci se chevauchent.

11. Dispositif de volatilisation de produit chimique selon la revendication 2,
dans lequel la distance entre les fibres de rétention de produit chimique en maillage (2) sur les deux côtés est 1,0 à 10,0 mm.
